# EUROPÄISCHE PATENTSCHRIFT

(11) **EP 2 138 681 B1**
(45) Veröffentlichungstag und Bekanntmachung des Hinweises auf die Patenterteilung: **27.03.2019**
(21) Anmeldenummer: 08011654.4
(22) Anmeldetag: 27.06.2008
(51) Int. Cl.: F01N 3/021, F01N 3/025, F01N 3/035, F01N 3/20, F01N 3/28, F01N 9/00, F02B 37/02, B01D 53/94, B01J 35/00, B01J 23/44, B01J 29/68, F01N 3/023, F01N 13/00

(54) **Verfahren und Vorrichtung zur Reinigung von Dieselabgasen**
Method and device for cleaning diesel exhaust gases
Procédé et dispositif de nettoyage de gaz d'échappement diesel

(43) Veröffentlichungstag der Anmeldung: 30.12.2009
(60) Teilanmeldung: 18206151.5
(73) Patentinhaber: Umicore AG & Co. KG, 63457 Hanau-Wolfgang (DE)
(72) Erfinder: Spurk, Paul, 64331 Weiterstadt (DE); Pfeifer, Marcus, 42719 Solingen (DE); Noack, Hendrik-David, 63457 Hanau (DE)

(56) Entgegenhaltungen:
- EP-A- 0 381 236
- EP-A- 1 072 763
- WO-A-97/36676
- WO-A-98/28070
- WO-A-2005/025725
- WO-A-2007/069994

## Beschreibung

Die Erfindung betrifft ein Verfahren und eine Vorrichtung zur Reinigung von Abgasen gemäß dem Oberbegriff des Anspruchs 1, bzw. 4, die durch einen Dieselmotor mit Ladeturbine erzeugtwerden, wobei die Vorrichtung in Strömungsrichtung des Abgases eine Dosierungsvorrichtung für ein Reduktionsmittel, einen SCR-Katalysator, einen Oxidationskatalysator und ein Dieselpartikelfilter enthält.

Das Rohabgas von Dieselmotoren enthält neben Kohlenmonoxid CO, Kohlenwasserstoffen HC und Stickoxiden NOₓ einen relativ hohen Sauerstoffgehalt von bis zu 15 Vol.-%. Außerdem sind Partikelemissionen enthalten, die überwiegend aus Rußrückständen und gegebenenfalls organischen Agglomeraten bestehen und aus einer partiell unvollständigen Kraftstoffverbrennung im Zylinder herrühren.

Die Einhaltung künftig in Europa, Nordamerika und Japan geltender gesetzlicher Abgasgrenzwerte für Dieselfahrzeuge erfordert die gleichzeitige Entfernung von Partikeln und Stickoxiden aus dem Abgas. Die Schadgase Kohlenmonoxid und Kohlenwasserstoffe können aus dem mageren Abgas durch Oxidation an einem geeigneten Oxidationskatalysator leicht unschädlich gemacht werden. Zur Entfernung der Partikelemissionen sind Dieselpartikelfilter mit und ohne zusätzliche katalytisch aktive Beschichtung geeignete Aggregate. Die Reduktion der Stickoxide zu Stickstoff ("Entstickung" des Abgases) ist wegen des hohen Sauerstoffgehaltes schwieriger. Ein bekanntes Verfahren ist die selektive katalytische Reduktion (Selective Catalytic Reduktion SCR) der Stickoxide an einem geeigneten Katalysator, kurz SCR-Katalysator. Dieses Verfahren gilt gegenwärtig für die Entstickung von Dieselmotorenabgasen als bevorzugt. Die Verminderung der im Abgas enthaltenden Stickoxide erfolgt im SCR-Verfahren unter Zuhilfenahme eines aus einer externen Quelle in den Abgasstrang eindosierten Reduktionsmittels. Als Reduktionsmittel wird bevorzugt Ammoniak oder eine Ammoniak freisetzende Verbindung wie beispielsweise Harnstoff oder Ammoniumcarbamat eingesetzt. Das gegebenenfalls aus der Vorläuferverbindung in situ erzeugte Ammoniak reagiert am SCR-Katalysator mit den Stickoxiden aus dem Abgas in einer Komproportionierungsreaktion zu Stickstoff und Wasser.

Derzeit ist, um den aufkommenden gesetzlichen Vorgaben gerecht zu werden, eine Kombination der verschiedenen Abgasreinigungsaggregate unumgänglich. Eine Vorrichtung zur Reinigung von Dieselmotorenabgasen muß mindestens einen oxidationsaktiven Katalysator und zur Entstickung einen SCR-Katalysator mit vorgeschalteter Einbringvorrichtung für Reduktionsmittel (bevorzugt Ammoniak oder Harnstofflösung) und externer Reduktionsmittelquelle (beispielsweise einen Zusatztank mit Harnstofflösung oder einen Ammoniakspeicher) enthalten. Falls es durch Optimierung der motorischen Verbrennung nicht gelingt, die Partikelemissionen so gering zu halten, daß diese über dem Oxidationskatalysator durch direkte Oxidation mit Sauerstoff entfernt werden können, ist zusätzlich der Einsatz eines Partikelfilters notwendig.

Entsprechende Abgasreinigungssysteme sind bereits beschrieben worden; einige befinden sich derzeit in der praktischen Erprobung.

So beschreibt die EP-B-1 054 722 ein System zur Behandlung von NOₓ- und Partikelhaltigen Dieselabgasen, worin ein Oxidationskatalysator einem Partikelfilter vorgeschaltet ist. Abströmseitig zum Partikelfilter sind eine Reduktionsmittelquelle und eine Dosiereinrichtung für das Reduktionsmittel, sowie ein SCR-Katalysator angeordnet.

In US 2007/0044456 wird ein Abgasnachbehandlungssystem offenbart, das anströmseitig zu einem Harnstoff-SCR-Katalysator (bevorzugt Übergangsmetall/Zeolith-Formulierung mit einem NOₓ-Konvertierungsoptimum im Temperaturbereich zwischen 200 und 500°C) einen Oxidationskatalysator (Platin-haltiger Edelmetallkatalysator) und abströmseitig zum SCR-Katalysator einen Dieselpartikelfilter enthält. Eine Dosiervorrichtung für Harnstoff ist zwischen dem Oxidationskatalysator und dem SCR-Katalysator angeordnet.

Beiden Systemen ist gemeinsam, daß das durch den Motor erzeugte Rohabgas im ersten Nachbehandlungsschritt über einen Oxidationskatalysator geleitet wird. Die Erfinder haben nun festgestellt, daß derartige Systeme, die als erste Abgasnachbehandlungsstufe einen Oxidationskatalysator enthalten, ohne Einführung zusätzlicher Hilfmaßnahmen nicht geeignet sind, um das Abgas von Dieselmotoren neuester Bauart, wie sie beispielsweise für EU-VI-Fahrzeuge vorgesehen sind, soweit aufzureinigen, daß die vorgeschriebenen Stickoxidemissionsgrenzwerte eingehalten werden können. Aus WO 97/36676 ist ein Verfahren und eine Vorrichtung zur Reinigung von Abgasen bekannt, die eine Frinepritsvorrichtung für harnstoff vor Turbolader und einen abstronseitig sitzenden SCR-Katelysatur aufwird.

Es ist Aufgabe der vorliegenden Erfindung, ein Abgasreinigungssystem (Verfahren und Vorrichtung) zur Verfügung zu stellen, mit dem das Abgas von Dieselmotoren neuester

Bauart, die eine Ladeturbine aufweisen, soweit aufgereinigt werden kann, daß auch die zukünftigen gesetzlichen Emissionsgrenzwerte ohne zusätzliche Hilfmaßnahmen eingehalten werden können.

Diese Aufgabe wird durch das Kenszeichen des Auspruchs 1, bzw 4. Die Abgase under von einem Dieselmotor mit Ladeturbine erzeugt und enthalten neben Kohlenmonoxid, Kohlenwasserstoffen und Partikeln Stickoxide mit einem NO₂/NOₓ-Verhältnis zwischen 0,3 und 0,7 wobei das Abgas über einen SCR-Katalysator zur Reduktion der Stickoxide zu Stickstoff, über einen Oxidationskatalysator zur Oxidation von Kohlenmonoxid und Kohlenwasserstoffen zu CO₂ und durch einen Dieselpartikelfilter zur Entfernung von Partikeln geleitet wird. Das Verfahren ist dadurch gekennzeichnet, daß als Reduktionsmittel für die SCR-Reaktion Harnstofflösung oder die Lösung einer anderen, wasserlöslichen, Ammoniak freisetzenden Verbindung verwendet und diese vor der Ladeturbine in den Abgasstrang eindosiert wird. Zur Durchführung des erfindungsgemäßen Verfahrens wird eine Vorrichtung zur Reinigung dieser Abgase bereitgestellt. Diese enthält in Strömungsrichtung des Abgases angeordnet eine Dosiervorrichtung für eine Reduktionsmittellösung aus einem Reduktionsmittelreservoir, einen SCR-Katalysator zur Reduktion von Stickoxiden, einen Oxidationskatalysator zur Oxidation von Kohlenmonoxid und Kohlenwasserstoffen und einen nachgechelteten Dieselpartikelfilter.

Das für den SCR-Katalysator optimale NO/NO₂-Verhältnis liegt für alle derzeit bekannten SCR-Katalysatoren um 1. Als NO₂/NOₓ-Verhältnis angegeben liegt das optimale Verhältnis zwischen 0,3 und 0,7. Ob dieses Verhältnis vor dem SCR-Katalysator in einem System nach EP-B-1 054 722 bzw. nach US 2007/0044456 erreicht wird, hängt von der Abgastemperatur und somit vom Betriebszustand des Motors und von der Aktivität des Oxidationskatalysators ab. Im Falle des in EP-B-1 054 722 beschriebenen Systems kommt als weitere Einflußgröße die Ausgestaltung und Rußbeladung des dem Oxidationskatalysator nachgeschalteten Dieselpartikelfilters hinzu.

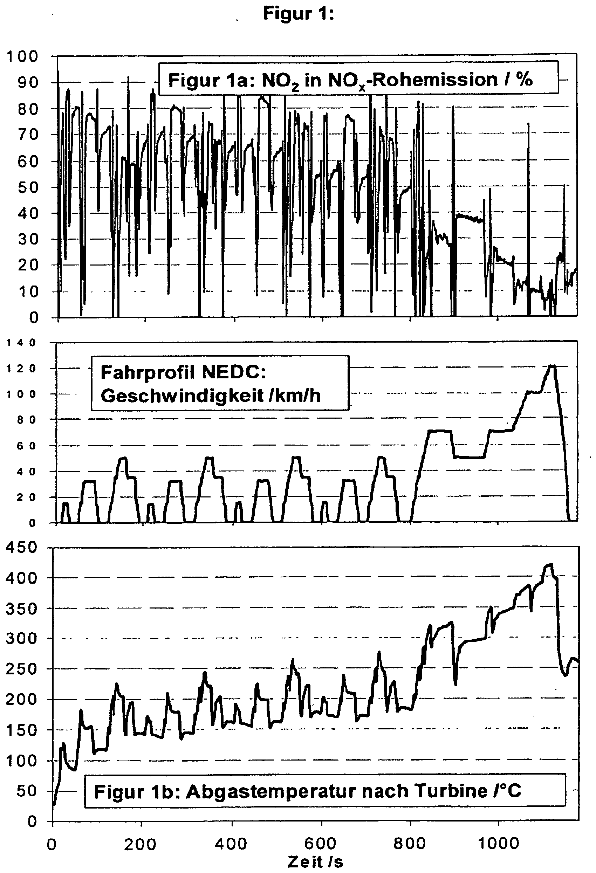

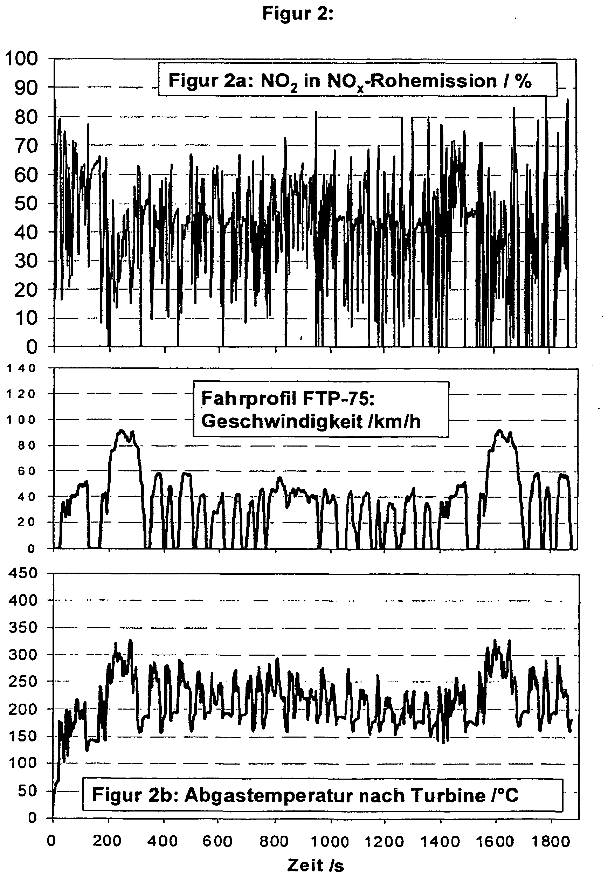

Dieselmotoren neuester Bauart unterscheiden sich von den bislang üblichen Dieselmotoren durch eine erheblich höhere Abgasrückführungsrate. Diese bedingt einen Anstieg des NO₂-Anteils im NOₓ der Rohemission auf bei gleichzeitig deutlicher Absenkung der mittleren Abgastemperatur. In vielen regulären Betriebspunkte liegt ein NO₂/NOₓ-Verhältnis von 0,3 bis 0,7 vor. Figur 1a zeigt beispielhaft den NO₂-Anteil im NOₓ der Rohemission eines entsprechenden Motors (4-Zylinder Common Rail Dieselmotor; Hubraum, 2,2 l) im europäischen Standardfahrzyklus New European Driving Cycle NEDC; Figur 1b zeigt das zugehörige Abgastemperaturprofil. In Figur 2 sind die relevanten Emissions- und Abgastemperaturdaten desselben Motors für den Betrieb im nordamerikanischen Standardfahrzyklus FTP-75 dargestellt (a: NO₂-Anteil in der Rohemission; b: Abgastemperaturniveau).

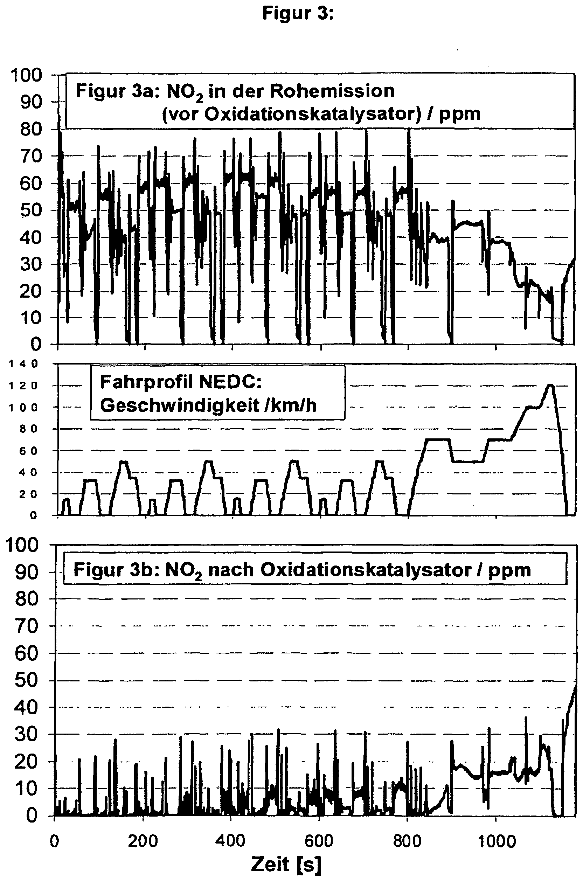

Die geänderten Rahmenbedingungen führen dazu, daß eine Überleitung des Rohabgases eines Dieselmotors neuester Bauart über einen Oxidationskatalysator in der ersten Abgasnachbehandlungsstufe nicht mehr, wie in EP-B-1 054 722 beschrieben, zu einer mindestens teilweisen Oxidation von NO zu NO₂ und somit zu einer Erhöhung des NO₂/NOₓ-Verhältnisses führt Die Erfinder haben vielmehr festgestellt, daß der Oxidationskatalysator unter den resultierenden Betriebsbedingungen als NO₂-vernichtendes Aggregat wirkt. Figur 3 zeigt einen Vergleich der NO₂-Konzentration im Abgas eines Dieselmotors neuester Bauart vor (Figur 3a) und nach (Figur 3b) Oxidationskatalysator. Es ist deutlich zu erkennen, daß der NO₂-Gehalt über dem Oxidationskatalysator deutlich vermindert wird. Diese NO₂-Reduktion geht jedoch nicht mit einer Entstickung des Abgases, d.h. mit einer signifikanten Verminderung des NOₓ-Gesamtgehaltes im Abgas einher. Da das über dem Oxidationskatalysator vemichtete NO₂ infolge des niedrigeren Abgastemperaturniveaus über dem nach EP-B-1 054 722 am Ende der Abgasanlage angeordneten SCR-Katalysator nicht mehr oxidativ nachgebildet werden kann, hat der nachgeschaltete SCR-Katalysator keine optimale Entstickungswirkung mehr. So kommt es in Niedriglast- und Teillastbetriebspunkten regelmäßig zu hohen NOₓ-Durchbrüchen; die künftig strengeren Stickoxidemissionsgrenzwerte werden überschritten.

Im erfindungsgemäßen Abgasreinigungssystem wird das von einem Dieselmotor mit Ladeturbine stammende Abgas zunächst durch Überleiten über einen SCR-Katalysator gezielt von Stickoxiden befreit. Das Abgas dieser neuen Motoren weist im Mittel ein nahezu optimales NO₂/NOₓ-Verhältnis von 0,3 bis 0,7 für die SCR-Reaktion auf, so daß in allen Betriebspunkten des Motors, auch in Kaltstart- und Niedriglastpunkten, in denen die Abgastemperatur unterhalb von 200°C liegt, optimale Entstickungsraten erzielt werden können. Als Reduktionsmittel in der SCR-Reaktion wird Harnstoff oder eine andere, wasserlösliche, Ammoniak freisetzende Verbindung verwendet. Diese Reduktionsmittellösung wird vor der Ladeturbine in den Abgasstrang eindosiert, so daß die Ladeturbine als Mischelement zur Homogenisierung von Reduktionsmittel und Abgas verwendet werden und die Hydrolysereaktion des Reduktionsmittels zu Ammoniak bedingt durch das an dieser Stelle erhöhte Temperaturniveau von mindestens 180°C in jedem Betriebspunkt des Motors sichergestellt werden kann.

Durch die genannten Maßnahmen wird eine effektive Entstickungsleistung in der ersten Abgasnachbehandlungsstufe des erfindungsgemäßen Systems gewährleistet. Dies und die Tatsache, daß das Dieselpartikelfilter am Ende der Abgasleitung und somit an der kältesten Stelle angeordnet ist, führen dazu, daß im erfindungsgemäßen System eine passive Regeneration des Dieselpartikelfilters, die auf dem in situ erfolgenden Abbrand von Ruß mit NO₂ bei Temperaturen oberhalb von 280°C erfolgt, nicht unterstützt wird. Demzufolge muß das Dieselpartikelfilter bei Überschreiten eines kritischen Abgasgegendruckwertes aktiv regeneriert werden. Dabei werden die zum Abbrand des auf dem Filter abgeschiedenen Rußes benötigten Temperaturen durch Kraftstoffnacheinspritzung in den Abgasstrang und katalytische Verbrennung des Kraftstoffs erzeugt. In einer bevorzugten Ausführungsform erfolgt die Kraftstoffnacheinspritzung anströmseitig zum SCR-Katalysator. Der eingespritzte Kraftstoff wird auf dem abströmseitig zum SCR-Katalysator angeordneten Oxidationskatalysator katalytisch verbrannt. Die resultierende Exotherme ist ausreichend, um die Temperatur im nachgeschalteten Dieselpartikelfilter auf Werte oberhalb der Rußzündtemperatur zu erhöhen. In einer alternativen Ausführungsform erfolgt die Kraftstoffnacheinspritzung zwischen Oxidationskatalysator und Dieselpartikelfilter. Die katalytische Verbrennung des Kraftstoffs kann dann beispielsweise auf einer oxidationskatalytisch aktiven Beschichtung erfolgen, die auf das Dieselpartikelfilter aufgebracht ist. Alternativ kann dem Dieselpartikelfilter ein zweiter Oxidationskatalysator, der als Heizkatalysator wirkt, unmittelbar vorgeschaltet werden. Die beiden letztgenannten Ausführungsformen haben den Vorteil, daß die zur aktiven Partikelfilterregeneration benötigte Kraftstoffmenge nicht als Kohlenwasserstoffbalast über den SCR-Katalysator geschleppt werden muß. Damit wird das Vergiftungsrisiko des SCR-Katalysators deutlich vermindert. Ferner können in solchen Ausführungsform sowohl die katalytisch aktive Beschichtung des Dieselpartikelfilters als auch die katalytisch aktive Beschichtung eines gegebenenfalls vorgeschalteten Heizkatalysators optimal auf die Erfordernisse der Partikelreinigung des Abgases und der Partikelfilterregeneration angepaßt werden, ohne Zielkonflikte mit anderen Abgasreinigungsaufgaben in Kauf nehmen zu müssen.

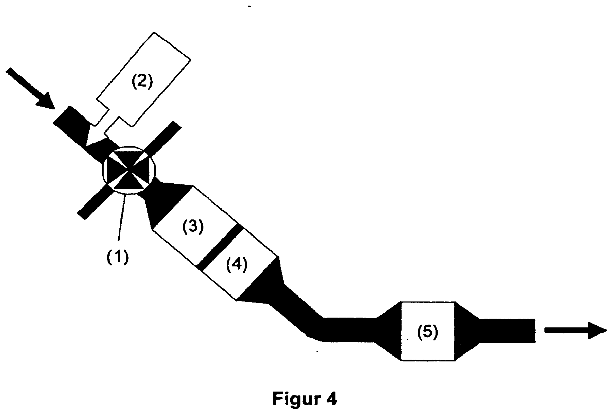

Figur 4 zeigt eine schematische Darstellung einer Vorrichtung gemäß Anspruch 6 zur Durchführung des beschriebenen Verfahrens. Das vom Dieselmotor neuester Bauart erzeugte Abgas, dessen Strömungsrichtung durch die Pfeile gekennzeichnet ist, enthält bei Eintritt in die Vorrichtung neben den üblichen Emissionen Kohlenmonoxid, Kohlenwasserstoffe und Partikel Stickoxide mit einem NO₂/NOₓ-Verhältnis zwischen 0,3 und 0,7. Noch vor Passieren der Ladeturbine (1) wird dem Abgas über eine Dosiervorrichtung eine Reduktionsmittellösung aus einem Reduktionsmittelreservoir (2) zugeführt. Als Reduktionsmittellösung wird bevorzugt Harnstofflösung oder die Lösung einer anderen, wasserlöslichen, Ammoniak freisetzenden Verbindung verwendet, die aus einem entsprechenden Tank über eine konventionelle Einspritzvorrichtung zugeführt wird. Beim Passieren der Ladeturbine erfolgt neben der Hydrolyse der Reduktionsmittellösung eine nahezu vollständige Homogenisierung von Reduktionsmittel und Abgas. Abströmseitig zur Ladeturbine ist in motornaher Position ein SCR-Katalysator (3) angeordnet, der die im Abgas enthaltenden Stickoxide mit dem aus der Hydrolyse der Reduktionsmittellösung erzeugten Ammoniak zu Stickstoff reduziert. Erst danach werden in einem abströmseitig angeordneten Oxidationskatalysator (4) Kohlenmonoxid (CO) und Kohlenwasserstoffe (HC) durch Aufoxidieren zu Kohlendioxid (CO₂) unschädlich gemacht. Gegebenenfalls im Abgas noch enthaltendes, im SCR-Katalysator unverbrauchtes Ammoniak wird im Oxidationskatalysator ebenfalls oxidativ entfernt. Um Temperaturverluste über die Abgasanlage so gering wie möglich zu halten und so möglichst hohe CO- und HC-Umsatzraten sicherzustellen, ist der Oxidationskatalysator bevorzugt ebenfalls motornah angeordnet, vorzugsweise im gleichen Gehäuse wie der SCR-Katalysator (3). Das dieses Gehäuse verlassende Abgas enthält neben unschädlichen Bestandteilen nur noch Partikel. Es strömt weiter zu einem aus BauraumGründen bevorzugt im Unterbodenbereich des Fahrzeugs anzuordnenden Dieselpartikelfilter (5), bei dessen Durchtritt die Partikel herausgefiltert werden, so daß am Ende der Anlage ein Abgas in die Umwelt entlassen wird, daß den gesetzlichen Anforderungen genügt.

Um die erfindungsgemäße Vorrichtung möglichst effektiv und mit einem hohen Abgasreinigungswirkungsgrad betreiben zu können, kommt neben der richtigen applikativen Auslegung der Wahl geeigneter Katalysatoren einige Bedeutung zu.

So ergibt sich aus der motomahen Anordnung des SCR-Katalysators am anströmseitigen Ende der Vorrichtung die Anforderung, daß der verwendete SCR-Katalysator eine möglichst hohe Vergiftungsresistenz gegenüber Kohlenwasserstoffen neben einer hinreichenden thermischen Alterungsstabilität bei Temperaturen von bis zu 800°C aufweisen sollte. Nicht alle konventionellen SCR-Katalysatortechnologien werden diesen Anforderungen gerecht. So neigen konventionelle zeolithische SCR-Katalysatoren, wie sie beispielsweise in US 4,961,917 beschrieben sind, aufgrund ihrer großen zeolithischen Porenweiten dazu, Kohlenwasserstoffe im zeolithischen Gerüst einzulagern, was zur Blockade von für die Funktionsweise dieser Katalysatoren wesentlichen Ammoniak-Speicherplätzen und katalytischen Übergangsmetall-Reaktionszentren führen und die Aktivität dieser Katalysatoren deutlich herabsetzen kann. Konventionelle Vanadiumpentoxid-basierte SCR-Katalysatoren weisen zumeist keine hinreichenden thermischen Alterungsstabilitäten auf.

Daher werden in der erfindungsgemäßen Vorrichtung bevorzugt Ceroxid-basierte SCR-Technologien eingesetzt, wie sie beispielsweise in WO 2008/049491 beschrieben sind. Erfindungsgemäß sind SCR-Katalysatoren basierend auf Übergangsmetall-ausgetauschten Zeolithverbindungen oder die zeolithähnlichen Materialien SAPO-34 oder SAPO-11, deren größte untere Kanalweite zwischen 0,26 und 0,42 nm beträgt, vorzugsweise deren größte untere Kanalweite einen Wert von 0,4 ± 0,01 nm nicht überschreitet. SCR-Katalysatoren dieses Typs enthalten bevorzugt Zeolithe ausgewählt aus der Gruppe bestehend aus Ferrierit, Chabazit, Erionit und Mischungen davon, welches einen Übergangsmetallgehalt von 0,1 bis 10 Gew.-% bezogen auf das Gewicht des Zeolithen bzw. des zeolithähnlichen Materials aufweist, wobei das Übergangsmetall besonders bevorzugt ausgewählt ist aus Eisen, Kupfer und Mischungen davon.

Für den abströmseitig zum SCR-Katalysator angeordneten Oxidationskatalysator ergibt sich infolge der Einbauposition, die sich vor allem durch ein kälteres Abgastemperaturniveau als in Vorrichtungen nach dem Stand der Technik üblich auszeichnet, die Anforderung, daß der Katalysator eine Zündtemperatur (Light off Temperatur) für die Kohlenwasserstoff- und Kohlenmonoxid-Oxidation von maximal 150°C aufweisen sollte. Um diese Anforderungen zu erfüllen, ist es vorteilhaft, im Oxidationskatalysator gegenüber Systemen nach dem Stand der Technik erhöhte Edelmetallgehalte einzusetzen. Da das den Oxidationskatalysator durchströmende Abgas in der erfindungsgemäßen Vorrichtung bereits entstickt ist, sind gute NO-Konversionsraten verzichtbar. Somit können mögliche Mehrkosten, die durch die insgesamt zu erhöhende Edelmetallmenge zunächst zu erwarten wären, dadurch kompensiert werden, daß anstelle der sonst üblichen hohen Anteile an Platin größere Mengen des kostengünstigeren Palladiums als katalytisch aktiver Komponente eingesetzt werden. Bevorzugt enthält ein in der erfindungsgemäßen Vorrichtung zum Einsatz kommender Oxidationskatalysator zwischen 0,35 und 7 Gramm pro Liter [g/L] Edelmetall bezogen auf das Katalysatorvolumen, besonders bevorzugt 3 bis 5 g/L. Das Edelmetall sollte ausgewählt sein aus der Gruppe bestehend aus Platin, Palladium, Rhodium, Iridium, Ruthenium und Mischungen davon. Bevorzugt wird Platin in Kombination mit Palladium eingesetzt, aus Kostengründen jedoch nicht Rhodium. Das Platin : Palladium-Verhältnis sollte zwischen 10 : 1 und 1 : 5 liegen, bevorzugt zwischen 8 : 1 und 1 : 1, besonders bevorzugt zwischen 5 : 1 und 2 : 1.

Um ein gutes Anspringverhalten des Oxidationskatalysators zu gewährleisten, ist es vorteilhaft, wenn der Oxidationskatalysator in Form einer katalytischen Beschichtung auf einem schnell durchwärmenden Tragkörper vorliegt. Als solche kommen beispielsweise metallische Wabenkörper oder keramischer Dünnwandwabenkörper (Wandstärke: 0,06 bis 0,1 Millimeter) mit Standardzelligkeiten (62 bis 124 Zellen pro Quadratzentimeter) in Frage.

Als Dieselpartikelfilter wird in der erfindungsgemäßen Vorichtung bevorzugt ein katalytisch beschichtetes Wandflußfiltersubstrat aus keramischem Material oder Siliciumcarbid eingesetzt. Die katalytische Beschichtung sollte so beschaffen sein, daß sie einerseits die Rußzündtemperatur so effektiv wie möglich herabsetzt, andererseits eine möglichst niedrige Zündtemperatur in der Kohlenwasserstoffoxidation zeigt, um bei der aktiven Partikelfilterregeneration gegebenenfalls im Filtereintritt vorhandene unverbrannte Kohlenwasserstoffe schnellstmöglich zu verbrennen und so zur Erzeugung der zum Erreichen der Rußzündtemperatur erforderlichen Exotherme so effektiv wie möglich beitragen zu können. Für den Regelbetrieb des Dieselpartikelfilters zwischen den aktiven Filterregenerationsphasen ist es weiterhin vorteilhaft, wenn die katalytische Beschichtung so beschaffen ist, das gegebenenfalls durch den Oxidationskatalysator durchbrechende Kohlenwasserstoffe und aus der Überoxidation von überschüsseigem Ammoniak resultierende Stickoxide, miteinander zu Stickstoff, Kohlendioxid und Wasser umgesetzt werden können (sog. "HC-DeNOx-Eigenschaften"). Um diese Funktionalitäten zu erreichen, enthält die katalytisch aktive Beschichtung vorzugsweise 0,15 bis 2 Gramm pro Liter [g/L] Edelmetall ausgewählts aus der Gruppe bestehend aus Platin, Palladium und Mischungen davon, bezogen auf das Volumen des Dieselpartikelfilters. Besonders bevorzugt sind 0,35 bis 1 g/L Edelmetall.

Beispielhaft kann eine erfindungsgemäße Vorrichtung ausgerüstet sein mit:
- einem SCR-Katalysator, V = 3,0 L, enthaltend einen Wabenkörper mit einer Zellzahl von 62 Zellen pro Quadratzentimeter und einer Zellwandstärke von 0,17 Millimeter, der mit einer katalytisch aktiven Beschichtung versehen ist enthaltend einen mit 5 Gew.-% Kupfer ausgetauschten Zeolithen des Ferrierit-Typs mit einer unteren Kanalweite von maximal 4,2 A;
- einem Oxidationskatalysator, V = 2,0 L, enthaltend einen keramischen Wabenkörper mit einer Zellzahl von 62 Zellen pro Quadratzentimeter und einer Zellwandstärke von 0,1 Millimeter, der mit einer katalytisch aktiven Beschichtung versehen ist, die 4 g/L Platin und Palladium im Verhältnis von 2 : 1, bezogen auf das Volumen des Oxidationskatalysators, auf einem Gemisch aus homogenem Siliciumdioxid-Aluminiumoxid-Mischoxid und γ-Aluminiumoxid mit einer aktiven Oberfläche von 200 m²/g (BET) enthält; und
- einem katalytisch aktivierten Dieselpartikelfilter mit einem Volumen von 4,0 L, enthaltend ein keramisches Wandflußfiltersubstrat mit einer Zellzahl von 48 Zellen pro Quadratzentimeter und einer Porosität von 50 %, in dessen Wände eine katalytisch aktive Beschichtung eingebracht ist, die einen Edelmetallgehalt von 0,9 g/L, bezogen auf das Volumen des Dieselpartikelfilters und ein Platin : Palladium-Verhaltnis von 1 : 1 aufweist.

Die erfindungsgemäße Vorrichtung eignet sich zur Reinigung der Abgase von Dieselfahrzeugen, insbesondere zur Reinigung der Abgase von Dieselpersonenkraftwagen, in denen Motoren mit Turbolader (Ladeturbine) und Abgasrückführungsvorrichtung eingesetzt werden.

## Patentansprüche

1. Verfahren zur Reinigung von Abgasen, die von einem Dieselmotor mit Ladeturbine (1) erzeugt werden und neben Kohlenmonoxid, Kohlenwasserstoffen und Partikeln Stickoxide mit einem NO₂/NOₓ-Verhältnis zwischen 0,3 und 0,7 enthalten, wobei das Abgas über einen SCR-Katalysator (3) zur Reduktion der Stickoxide zu Stickstoff, über einen Oxidationskatalysator (4) zur Oxidation von Kohlenmonoxid und Kohlenwasserstoffen zu CO₂ und durch einen Dieselpartikelfilter (5) zur Entfernung von Partikeln geleitet wird, wobei als Reduktionsmittel für die SCR-Reaktion Harnstofflösung oder die Lösung einer anderen, wasserlöslichen, Ammoniak freisetzenden Verbindung verwendet wird, welche vor der Ladeturbine (1) in den Abgasstrang eindosiert wird,
**dadurch gekennzeichnet,**
**dass** der Dieselpartikelfilter (5) nachgeschaltet ist und der SCR-Katalysator (3) eine oder mehrere Übergangsmetall-ausgetauschte Zeolithverbindungen oder die zeolithähnlichen Materialien SAPO-34 oder SAPO-11 mit einer größten unteren Kanalweite von 0,26 bis 0,42 nm enthält.

2. Verfahren nach Anspruch 1,
**dadurch gekennzeichnet,**
**dass** die Kraftstoffnacheinspritzung zur Regeneration des Dieselpartikelfilters (5) anströmseitig zum SCR-Katalysator (3) und die katalytische Verbrennung des Kraftstoffs auf dem abströmseitig zum SCR-Katalysator angeordneten Oxidationskatalysator (4) erfolgt.

3. Verfahren nach Anspruch 1,
**dadurch gekennzeichnet,**
**dass** die Kraftstoffnacheinspritzung zur Regeneration des Dieselpartikelfilters (5) zwischen Oxidationskatalysator (4) und Dieselpartikelfilter (5) und die katalytische Verbrennung des Kraftstoffs auf einer oxidationskatalytisch aktiven Beschichtung, die auf das Dieselpartikelfilter (5) aufgebracht ist, oder auf einem zweiten, dem Dieselpartikelfilter (5) unmittelbar vorgeschalteten, als Heizkatalysator wirkenden Oxidationskatalysator erfolgt.

4. Vorrichtung zur Reinigung von Abgasen, die von einem Dieselmotor mit Ladeturbine (1) erzeugt werden und neben Kohlenmonoxid, Kohlenwasserstoffen und Partikeln Stickoxide mit einem NO₂/NOₓ-Verhältnis zwischen 0,3 und 0,7 enthalten, enthaltend in Strömungsrichtung des Abgases angeordnet:
i) eine Dosiervorrichtung für eine Reduktionsmittellösung aus einem Reduktionsmittelreservoir,
ii) einen SCR-Katalysator (3) zur Reduktion von Stickoxiden,
iii) einen Oxidationskatalysator (4) zur Oxidation von Kohlenmonoxid und Kohlenwasserstoffen und
iv) ein Dieselpartikelfilter (5),
und wobei die Dosiervorrichtung (2) für Reduktionsmittellösung anströmseitig zur Ladeturbine angeordnet ist,
**dadurch gekennzeichnet,**
**dass** der Dieselpartikelfilter (5) nachgeschaltet ist und dass der SCR-Katalysator eine oder mehrere Übergangsmetall-ausgetauschte Zeolithverbindungen oder die zeolithähnlichen Materialien SAPO-34 oder SAPO-11 mit einer größten unteren Kanalweite von 0,26 bis 0,42 nm enthält.

5. Vorrichtung nach Anspruch 4,
**dadurch gekennzeichnet,**
**dass** SCR-Katalysator (3) und Oxidationskatalysator (4) in motornaher Position und im gleichen Gehäuse angeordnet sind.

6. Vorrichtung nach einem der Ansprüche 4 und/oder 5,
**dadurch gekennzeichnet,**
**dass** der Oxidationskatalysator (4) in Form einer katalytischen Beschichtung auf einem schnell durchwärmenden Tragkörper vorliegt und 0,35 bis 7 g/L Edelmetall bezogen auf das Katalysatorvolumen enthält, wobei das Edelmetall ausgewählt ist aus der Gruppe bestehend aus Platin, Palladium, Rhodium, Iridium, Ruthenium und Mischungen davon.

7. Vorrichtung nach Anspruch 6,
**dadurch gekennzeichnet,**
**dass** als Edelmetall Platin in Kombination mit Palladium eingesetzt wird und das Platin : Palladium-Verhältnis zwischen 10 : 1 und 1 : 5 liegt.

8. Vorrichtung nach einem der Ansprüche 4 bis 7 ,
**dadurch gekennzeichnet,**
**dass** das Dieselpartikelfilter (5) ein katalytisch beschichtetes Wandflußfiltersubstrat aus keramischem Material oder Siliciumcarbid ist, und die katalytisch aktive Beschichtung 0,15 bis 2 g/L Edelmetall ausgewählt aus der Gruppe bestehend aus Platin, Palladium und Mischungen davon, bezogen auf das Volumen des Dieselpartikelfilters, enthält.

9. Verwendung einer Vorrichtung nach einem der Ansprüche 4 bis 8 zur Reinigung der Abgase von Dieselfahrzeugen.

## Claims

1. Method for cleaning exhaust gases that are produced by a diesel engine with a load turbine (1) and contain nitrogen oxides with an NO₂/NOₓ ratio between 0.3 and 0.7 in addition to carbon monoxide, hydrocarbons, and particulates, wherein the exhaust gas is guided over an SCR catalyst (3) in order to reduce the nitrogen oxides to nitrogen, over an oxidation catalyst (4) in order to oxidize carbon monoxide and hydrocarbons to CO₂, and through a diesel particulate filter (5) in order to remove particulates, wherein urea solution or the solution of another aqueous, ammonia-releasing compound, which is fed into the exhaust gas stream upstream of the load turbine (1), is used as reducing agent for the SCR reaction,
**characterized in that**
the diesel particulate filter (5) is arranged downstream, and the SCR catalyst (3) contains one or more transition-metal-exchanged zeolite compounds or the zeolite-like materials, SAPO-34 or SAPO-11, with a maximum lower channel width of 0.26 to 0.42 nm.

2. Method according to claim 1,
**characterized in that**
the fuel post-injection for regenerating the diesel particulate filter (5) takes place upstream of the SCR catalyst (3), and the catalytic combustion of the fuel takes place on the oxidation catalyst (4) arranged downstream of the SCR catalyst.

3. Method according to claim 1,
**characterized in that**
the fuel post-injection for regenerating the diesel particulate filter (5) takes place between the oxidation catalyst (4) and the diesel particulate filter (5), and the catalytic combustion of the fuel takes place on an oxidation-catalytically-active coating applied to the diesel particulate filter (5) or on a second oxidation catalyst arranged directly upstream of the diesel particulate filter (5) and acting as a heated catalyst.

4. Device for cleaning exhaust gases that are produced by a diesel engine with a load turbine (1) and that contain nitrogen oxides with an NO₂/NOₓ ratio between 0.3 and 0.7 in addition to carbon monoxide, hydrocarbons, and particulates, containing, arranged in the flow direction of the exhaust gas:
i) a metering device for a reducing agent solution from a reducing agent reservoir,
ii) an SCR catalyst (3) for reducing nitrogen oxides,
iii) an oxidation catalyst (4) for oxidizing carbon monoxide and hydrocarbons, and
iv) a diesel particulate filter (5),
and wherein the metering device (2) for the reducing agent solution is arranged upstream of the load turbine,
**characterized in that**
the diesel particulate filter (5) is arranged downstream, and the SCR catalyst contains one or more transition-metal-exchanged zeolite compounds or the zeolite-like materials, SAPO-34 or SAPO-11, with a maximum lower channel width of 0.26 to 0.42 nm.

5. Device according to claim 4,
**characterized in that**
the SCR catalyst (3) and the oxidation catalyst (4) are arranged in a position close to the engine and in the same housing.

6. Device according to any one of claims 4 and/or 5,
**characterized in that**
the oxidation catalyst (4) is provided in the form of a catalytic coating on a quickly, thoroughly heating support body and contains 0.35 to 7 g/L precious metal in relation to the catalyst volume, wherein the precious metal is selected from the group consisting of platinum, palladium, rhodium, iridium, ruthenium, and mixtures thereof.

7. Device according to claim 6,
**characterized in that**
platinum in combination with palladium is used as precious metal, and the platinum : palladium ratio is between 10 : 1 and 1 : 5.

8. Device according to any one of claims 4 through 7,
**characterized in that**
the diesel particulate filter (5) is a catalytically-coated wall-flow filter substrate made of ceramic material or silicon carbide, and the catalytically-active coating contains 0.15 to 2 g/L precious metal selected from the group consisting of platinum, palladium, and mixtures thereof in relation to the volume of the diesel particulate filter.

9. Use of a device according to any one of claims 4 through 8 for cleaning the exhaust gases of diesel vehicles.

## Revendications

1. Procédé de purification de gaz d'échappement, qui sont produits par un moteur diesel équipé d'un turbocompresseur (1) et qui contiennent, en plus de monoxyde de carbone, d'hydrocarbures et de particules, des oxydes d'azote présentant un rapport NO₂/NOₓ entre 0,3 et 0,7, dans lequel le gaz d'échappement est guidé sur un catalyseur de réduction catalytique sélective (SCR) (3) pour la réduction des oxydes d'azote en azote, sur un catalyseur d'oxydation (4) pour l'oxydation du monoxyde de carbone et des hydrocarbures en CO₂ et à travers un filtre à particules diesel (5) pour l'élimination des particules, dans lequel, en guise d'agent de réduction pour la réaction SCR, une solution d'urée ou la solution d'un autre composé soluble dans l'eau libérant de l'ammoniac est utilisée, qui est dosée en amont du turbocompresseur (1) dans la ligne d'échappement,
**caractérisé**
**en ce que** le filtre à particules diesel (5) est branché en aval et le catalyseur SCR (3) contient un ou plusieurs composés zéolithiques à métal de transition échangé ou les matériaux similaires à la zéolithe SAPO-34 ou SAPO-11 d'une largeur de canal inférieur la plus importante de 0,26 à 0,42 nm.

2. Procédé selon la revendication 1,
**caractérisé**
**en ce que** la post-injection de carburant pour la régénération du filtre à particules diesel (5) est effectuée en amont du catalyseur SCR (3) et la combustion catalytique du carburant est effectuée sur le catalyseur d'oxydation (4) placé en aval du catalyseur SCR.

3. Procédé selon la revendication 1,
**caractérisé**
**en ce que** la post-injection de carburant pour la régénération du filtre à particules diesel (5) est effectuée entre le catalyseur d'oxydation (4) et le filtre à particules diesel (5) et la combustion catalytique du carburant est effectuée sur un revêtement à action d'oxydation catalytique, qui est appliqué sur le filtre à particules diesel (5) ou sur un deuxième catalyseur d'oxydation fonctionnant comme un catalyseur de chauffage branché directement en amont du filtre à particules diesel (5).

4. Dispositif pour la purification de gaz d'échappement, qui sont produits par un moteur diesel équipé d'un turbocompresseur (1) et qui contiennent, en plus de monoxyde de carbone, d'hydrocarbures et de particules, des oxydes d'azote présentant un rapport NO₂/NOₓ entre 0,3 et 0,7, comprenant, disposés dans le sens d'écoulement du gaz d'échappement :
i) un dispositif de dosage pour une solution d'agent de réduction provenant d'un réservoir d'agent de réduction,
ii) un catalyseur SCR (3) pour la réduction des oxydes d'azote,
iii) un catalyseur d'oxydation (4) pour l'oxydation du monoxyde de carbone et des hydrocarbures et
iv) un filtre à particules diesel (5),
et dans lequel le dispositif de dosage (2) pour la solution d'agent de réduction est disposé en amont du turbocompresseur,
**caractérisé**
**en ce que** le filtre à particules diesel (5) est branché en aval et **en ce que** le catalyseur SCR contient un ou plusieurs composés zéolithiques à métal de transition échangé ou les matériaux similaires à la zéolithe SAPO-34 ou SAPO-11 présentant une largeur de canal inférieur la plus importante de 0,26 à 0,42 nm.

5. Dispositif selon la revendication 4,
**caractérisé**
**en ce que** le catalyseur SCR (3) et le catalyseur d'oxydation (4) sont placés dans une position proche du moteur et dans le même boîtier.

6. Dispositif selon l'une quelconque des revendications 4 et/ou 5,
**caractérisé**
**en ce que** le catalyseur d'oxydation (4) se trouve sous la forme d'un revêtement catalytique sur un corps de support à chauffage rapide et contient de 0,35 à 7 g/l de métal noble par rapport au volume du catalyseur, le métal noble étant choisi dans le groupe constitué par le platine, le palladium, le rhodium, l'iridium, le ruthénium et leurs mélanges.

7. Dispositif selon la revendication 6,
**caractérisé**
**en ce que** le platine est utilisé en association avec le palladium en guise de métal noble et le rapport platine:palladium se trouve entre 10:1 et 1:5.

8. Dispositif selon l'une quelconque des revendications 4 à 7,
**caractérisé**
**en ce que** le filtre à particules diesel (5) est un substrat à parois filtrantes à revêtement catalytique en matériau céramique ou en carbure de silicium et le revêtement à action catalytique contient 0,15 à 2 g/l de métal noble choisi dans le groupe constitué par le platine, le palladium et leurs mélanges, par rapport au volume du filtre à particules diesel.

9. Utilisation d'un dispositif selon l'une quelconque des revendications 4 à 8, pour la purification de gaz d'échappement de véhicules diesel.
